# EUROPÄISCHE PATENTSCHRIFT

(11) **EP 1 675 541 B1**
(45) Veröffentlichungstag und Bekanntmachung des Hinweises auf die Patenterteilung: **02.05.2007**
(21) Anmeldenummer: 04790722.5
(22) Anmeldetag: 21.10.2004
(51) Int. Cl.: A61F 13/06

(54) **BANDAGEN-EINLAGEN/PASSTEIL-VERBUND**
BANDAGE INSERT/INSET COMPOSITE
COMPOSITE D'INSERTION OU D'AJUSTAGE DE BANDAGE

(30) Priorität: 21.10.2003 DE 20316109 U
(43) Veröffentlichungstag der Anmeldung: 05.07.2006
(73) Patentinhaber: Lohrer, Heinz, 65835 Liederbach (DE)
(72) Erfinder: Lohrer, Heinz, 65835 Liederbach (DE)
(74) Vertreter: Zeitler, Giselher
(86) Internationale Anmeldenummer: PCT/EP2004/011920
(87) Internationale Veröffentlichungsnummer: WO 2005/039466

(56) Entgegenhaltungen:
- WO-A-01/43677
- DE-U1- 9 417 221
- GB-A- 286 132
- US-A- 1 538 026
- US-A- 1 577 203
- US-A- 1 684 948
- US-A1- 2002 066 456

## Beschreibung

Die Erfindung betrifft eine elastische Bandage in Hülsen- bzw. Röhrenform für verletzte bzw. verletzungsanfällige Gliedmaßen gemäß dem Oberbegriff des Anspruchs 1.

Derartige Bandagen, die in Hülsen- bzw. Röhrenform ausgestaltet sind, dienen üblicherweise zur Korrektur, Stützung und/oder Entlastung von verletzten bzw. verletzungsanfälligen Gliedmaßen.

Stattdessen oder zusätzlich hierzu kommen für denselben oder einen ähnlichen Zweck auch orthopädische bzw. orthetische Elemente zur Anwendung, wie beispielsweise Bettungs-, Entlastungs-, Stütz- und/oder Korrektureinlagen oder -passteile. Ein besonders häufiges Anwendungsgebiet sind hierbei Sportschuheinlagen, bei denen das Hauptproblem darin besteht, dass sie sicher im Schuh stabilisiert sind, um ein Verrutschen zu verhindern und um damit einen sicheren Kraftschluss zwischen Fuß und Schuh zu gewährleisten.

Es ist bisher noch nicht oder nur in unbefriedigender Weise gelungen, derartige orthopädische bzw. orthetische Elemente schnell und problemlos an der gewünschten Stelle des betreffenden Gliedmaßes zu positionieren und dabei gleichzeitig die Gewissheit zu haben, dass die gewünschte präzise Positionierung über einen längeren Gebrauch beibehalten wird.

Das Dokument US-A-1 538 026 beschreibt eine elastische Bandage gemäß dem Oberbegriff des Anspruchs 1.

Der Erfindung liegt daher die Aufgabe zugrunde, eine Vorrichtung zu schaffen, mit der es zur Beseitigung der geschilderten Nachteile möglich ist, das medizinisch gebotene orthopädische bzw. orthetische Element schnell und problemlos ohne Beanspruchung eines zu großen Volumens an der gewünschten Stelle des Gliedmaßes zu positionieren und gleichzeitig zu gewährleisten, dass die gewünschte individuelle präzise Positionierung stets beibehalten wird.

Die Merkmale der zur Lösung in dieser Aufgabe geschaffenen Erfindung ergeben sich aus Anspruch 1, insbesondere den Merkmalen des Kennzeichenteils.

Die Erfindung wird im folgenden anhand der Zeichnung näher erläutert. Diese zeigt in:
- Fig. 1: die elastische Bandage gemäß der Erfindung mit einer außenseitigen Tasche zur Halterung einer Stütze;
- Fig. 2: eine abgewandelte Ausführungsform hiervon in Form einer flügelartigen Lasche in Rechteckform und
- Fig. 3: in Dreieckform;
- Fig. 4: eine weitere Ausführungsform der Halterung in Form einer Klettverschlussfläche, die mittels eines entsprechend ausgebildeten Flächenstückes abdeckbar ist,
- Fig. 5: eine weitere Ausführungsform der Halterung im Längsschnitt und
- Fig. 6: im Querschnitt gemäß Linie VI-VI nach Fig. 5.

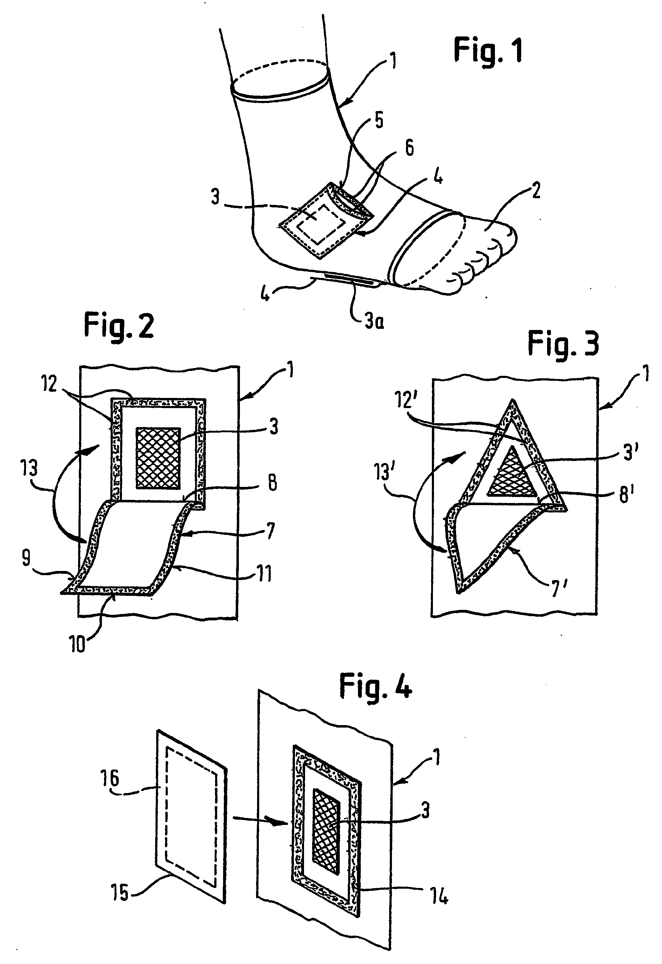

Wie aus Fig. 1 ersichtlich, ist die dargestellte elastische Bandage 1 in Hülsen- bzw. Röhrenform ausgestaltet und zur Anwendung an einem verletzten bzw. verletzungsanfälligen Fuß 2 und Sprunggelenk vorgesehen.

Eine zu diesem Zweck orthopädisch verordnete Stütze 3 in Form einer Einlage, einer Pelotte oder eines Passteiles soll auf der Höhe des Fußknöchels zu dessen Stützung angeordnet werden, und zwar ohne die Gefahr eines Verrutschens, d.h. also in elastischer Fixierung. Zu diesem Zweck weist die Bandage 1 außenseitig eine Halterung in Form einer seitlichen Tasche 4 auf, in welche die Stütze 3 einsteckbar ist.

Eine solche Tasche 4 ist auch an der Sohlenseite der Bandage 1 vorgesehen, in die eine Einlage 3a lösbar einsteckbar und somit dort sicher sowie präzise positionierbar ist.

Wie ersichtlich, ist die Tasche 4 an ihrem einen Ende 5 offen ausgebildet, jedoch mit ihren restlichen drei Rändern in geeigneter Weise an der Bandage 1 befestigt, beispielsweise mittels eines elastischen Fadens angenäht.

Das obere offene Ende 5 der Tasche 4 ist verschließbar ausgebildet. Zu diesem Zweck ist beim dargestellten Ausführungsbeispiel ein Klettverschluss 6 vorgesehen.

Bei der abgewandelten Ausführungsform der Halterung gemäß Fig. 2 ist eine flügelartige Lasche 7 vorgesehen. Diese kann als "dreiseitig offene Tasche" angesehen werden und ist, wie dargestellt, mit ihrem unteren Rand 8 an der Bandage 1 befestigt, während sie mit ihren drei anderen offenen Rändern 9, 10, 11 an der Bandage 1 festlegbar ist. Zu diesem Zweck weist die Bandage 1 einen entsprechend komplementär ausgebildeten Festlegungsbereich 12 auf, der - im Zusammenwirken mit den Laschenrändern 9, 10, 11 - beim dargestellten Ausführungsbeispiel gleichfalls als Klettverschluss ausgebildet ist.

Während bei der Ausführungsform gemäß Fig. 2 die flügelartige Lasche 7 als rechtwinkliges Flächenstück ausgebildet ist, ist bei der Ausführungsform gemäß Fig. 3 die Lasche 7' ein dreieckförmiges Flächenstück, wobei ansonsten eine entsprechende Ausgestaltung wie bei Fig. 2 vorgesehen ist.

Wie durch die Pfeile 13 bzw. 13' angedeutet, ist die jeweilige flügelartige Lasche 7 bzw. 7' dann, wenn die betreffende Einlage bzw. das betreffende Passteil 3 an die Außenseite der Bandage 1 angedrückt gehalten wird, in Pfeilrichtung nach oben zu klappen und mit den Innenseiten ihrer Ränder 9, 10, 11 in Kontakt mit dem entsprechenden Festlegungsbereich 12 bzw. 12' zu bringen, so dass dann hierdurch, ähnlich wie bei der Tasche 4 der Ausführungsform gemäß Fig. 1, die Einlage bzw. das Passteil 3 bzw. 3' sicher an der Bandage 1 in der gewünschten Position fixiert ist.

Bei der abgewandelten Ausführungsform gemäß Fig. 4 ist zur Halterung an der Bandage außenseitig eine Klettverschlussfläche 14 vorgesehen, die durch ein entsprechend komplementär ausgebildetes Flächenstück 15 zur Halterung des Passteiles 3 abdeckbar ist. Wie ersichtlich, ist diese Klettverschlussfläche 14 als an der Bandage 1 vorgesehener umlaufender Klettverschlussrand ausgebildet, der mit einem an der Innenseite des Flächenstückes vorgesehenen, entsprechend ausgebildeten Klettverschlussrand zusammenwirkt.

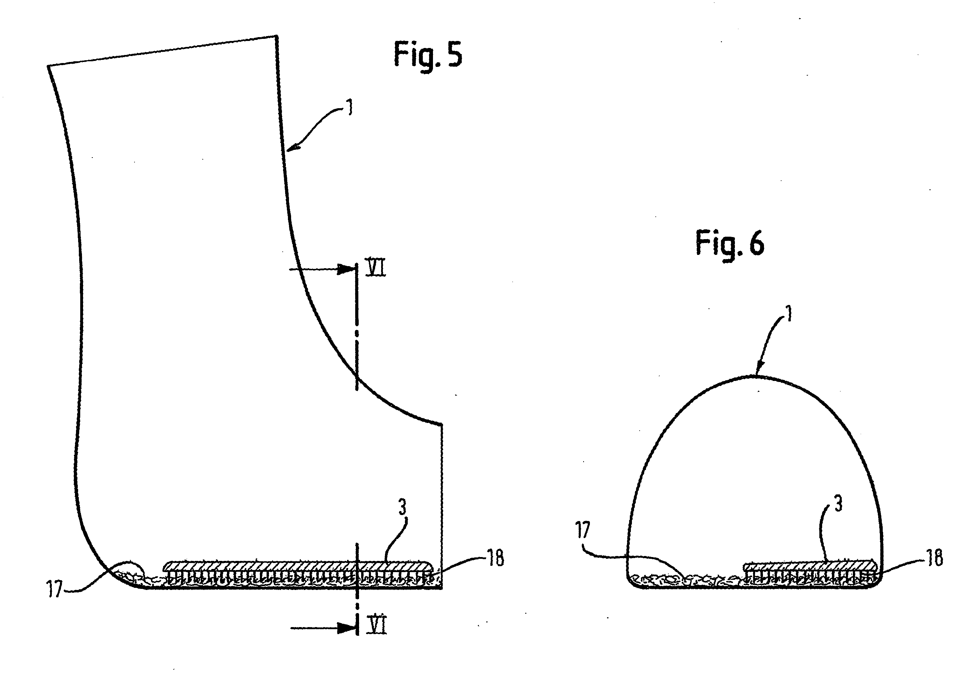

Wie schließlich aus Fig. 5 und 6 ersichtlich, ist bei dieser dargestellten Ausführungsform die Halterung innenseitig an der Bandage 1 angebracht und in Form einer Klettverschlussfläche 17 ausgebildet, die sich über den gesamten Fußsohlenbereich erstreckt. An dieser Klettverschlussfläche 17 kann an einer beliebigen gewünschten Stelle das Passteil 3 lösbar direkt festgelegt werden. Zu diesem Zweck weist das Passteil an seiner einen Seite, d.h. also an seiner "Unterseite", gleichfalls eine Klettverschlussfläche 18 auf, die in der üblichen Weise komplementär ausgebildet ist und mit der bandagenseitigen Klettverschlussfläche 17 zur lösbaren, gleichwohl sicheren und präzisen Halterung des Passteils 3 zusammenwirkt.

Hinsichtlich vorstehend nicht im einzelnen näher erläuterter Merkmale der Erfindung wird im übrigen ausdrücklich auf die Ansprüche sowie die Zeichnung verwiesen.

## Patentansprüche

1. Elastische Bandage in Hülsen- bzw. Röhrenform für verletzte bzw. verletzungsanfällige Gliedmaßen,
wobei
die Bandage (1) an ihrer Innenfläche eine Halterung (17) zur lösbaren Festlegung von orthopädischen bzw. orthetischen Elementen (3), wie Korrektur-, Stütz-, Entlastungs- und/oder Bettungseinlagen oder -passteilen, insbesondere Schuheinlagen, aufweist, **dadurch gekennzeichnet, dass**
die Halterung als eine an der Bandage (1) innenseitig angebrachte Klettverschlussfläche (17) ausgebildet ist, die sich über den gesamten Fußsohlenbereich erstreckt, und
an dieser Klettverschlussfläche (17) das Passteil (3) mittels einer an seiner einen Fläche angebrachten komplementären Klettverschlussfläche (18) an einer beliebigen gewünschten Stelle lösbar direkt festlegbar ist.

## Claims

1. Elastic bandage in sleeve-like or tubular form for limbs which are injured or prone to injury, the bandage (1) having on its inside surface a mounting (17) for the detachable fastening in place of orthopaedic or orthotic elements (3) such as corrective, supporting, relieving and/or cushioning inserts or fitted parts, and in particular arch supports, **characterised in that** the mounting is in the form of an area of hook-and-loop fastener (17) which is attached to the inside of the bandage (1) and which extends over the entire region occupied by the sole of the foot, and the fitted part (3) can be detachably fastened directly to any desired point on this area of hook-and-loop fastener (17) by means of a complementary area of hook-and-loop fastener (18) attached to one of its faces.

## Revendications

1. Bandage élastique sous forme de manchon ou de tube pour des membres blessés ou menacés par des blessures,
dans lequel
le bandage (1) comporte sur sa surface intérieure une monture (17) pour fixer de façon détachable des éléments orthopédiques (3) ou orthétiques (3), comme des inserts ou des pièces ajustées de correction, de soutien, de décharge et/ou de matelassage, en particulier des inserts de chaussures,
**caractérisé en ce que**
la monture est réalisée sous la forme d'une surface agrippante (17) appliquée du côté intérieur du bandage (1), qui s'étend sur la totalité de la région de la plante du pied, et
la pièce ajustée (3) est susceptible d'être fixée à un emplacement désiré quelconque directement et de façon détachable sur cette surface agrippante (17) au moyen d'une surface agrippante complémentaire (18) appliquée sur sa surface.
